# EUROPEAN PATENT APPLICATION

(11) **EP 4 789 658 A1**
(43) Date of publication of application: **12.08.2026**
(21) Application number: 24874163.9
(22) Date of filing: 02.10.2024
(51) Int. Cl.: A61K 9/08, A61K 9/00, A61K 47/12, A61K 47/18, A61K 47/40, A61K 47/36, A61K 31/498, A61P 27/02

(54) **OPHTHALMIC COMPOSITION COMPRISING BRIMONIDINE**

(30) Priority: 05.10.2023 KR 20230132468; 26.06.2024 KR 20240083850
(71) Applicant: Taejoon Pharmaceutical Co., Ltd., Seoul 04401 (KR)
(72) Inventor: LEE, Joon Youb, Seoul 04401 (KR); RYU, Sang Rok, Suwon-si Gyeonggi-do 16222 (KR); GO, Hee Kyoung, Suwon-si Gyeonggi-do 16516 (KR)
(74) Representative: HGF
(86) International application number: PCT/IB2024/059621
(87) International publication number: WO 2025/074260

(57) **Abstract**

The present invention relates to an ophthalmic composition comprising brimonidine or a salt thereof, citrate or a hydrate thereof, and trometamol, and having excellent stability and therapeutic effects.

## Description

### Technical Field

The present invention relates to an ophthalmic composition including brimonidine or a salt thereof.

### Background

Brimonidine is an alpha-2-adrenergic agonist which is a drug that reduces an intraocular pressure of patients with glaucoma and/or ocular hypertension.

As a representative ophthalmic preparation containing brimonidine, there is Alphagan^{®}-P (Allergan, Inc.) containing 0.15% brimonidine tartrate, which is prescribed to be administered three times a day.

Due to the nature of the ophthalmic preparation, it is desirable to have a pH in a biocompatible range capable of minimizing irritation to an eye and it is necessary to have storage stability such that no discoloration or precipitation occurs. However, since brimonidine has a property that solubility thereof rapidly decreases under a condition higher than pH 7, it may be difficult to formulate brimonidine into an aqueous solution having a transparent apperance at a pH of more than 7 and a pH of 8 or less as a biocompatible range.

An ophthalmic suspension has a difficulty in being used by shaking in order to disperse and administer a drug, may cause a feeling of irritation or blurred vision during administration, and may cause local pain. Thus, it is also necessary for ophthalmic preparations containing brimonidine to be made into an aqueous solution having a transparent apperance and to stably maintain the apperance.

Meanwhile, in order to secure a further increased therapeutic effect and convenience of intake in the ophthalmic preparation, it may be considered to increase a content of active ingredients. However, brimonidine is hardly dissolved within a biocompatible pH range and produces more impurities under a pH condition higher than pH 7, and thus there is a limit to the development of a transparent ophthalmic preparation containing a high concentration of brimonidine having excellent stability while maintaining biocompatibility.

### Related Art Reference

### Patent Documents

(Patent Document 1) Korean Laid-open Patent Publication No. 10-2003-0017500

### Description of the Invention

### Technical Problem

The present invention may provide an ophthalmic composition including brimonidine or a salt thereof, which includes brimonidine stably dissolved therein, may be safely administered to an eye, has a small amount of impurities produced during storage, and may be maintained in a stable state without precipitation and a change in properties during storage.

### Technical Solution

An ophthalmic composition according to the present invention may include: brimonidine or a salt thereof; citrate or a hydrate thereof; and trometamol.

In embodiments of the present invention, "brimonidine" may refer to a compound having a chemical name of 5-bromo-6-(2-imidazolidinylideneamino)quinoxaline.

In embodiments of the present invention, the salt of brimonidine may include brimonidine tartrate.

In embodiments of the present invention, the ophthalmic composition may include brimonidine or the salt thereof in an amount of 0.2 w/v% or more or 0.3 w/v% or more, and in an amount of 10 w/v% or less, 5 w/v% or less, 1 w/v% or less, 0.9 w/v% or less, 0.8 w/v% or less, 0.7 w/v% or less, 0.6 w/v% or less, or 0.5 w/v% or less based on the total volume of the ophthalmic composition. For example, the ophthalmic composition may include brimonidine or the salt thereof in an amount of 0.2 to 10 w/v%, 0.2 to 5 w/v%, 0.2 to 1 w/v%, 0.3 to 1 w/v%, 0.4 to 1 w/v%, 0.2 to 0.5 w/v%, 0.3 to 0.5 w/v%, or 0.3 to 0.4 w/v%.

In one embodiment, the ophthalmic composition may include brimonidine or the salt thereof in an amount of 0.2 w/v% or more based on the total volume of the ophthalmic composition. As one example, the ophthalmic composition may include brimonidine or the salt thereof in an amount of 0.2 to 1 w/v%, 0.3 to 1 w/v%, or 0.2 to 0.5 w/v%.

In one embodiment, the ophthalmic composition may include brimonidine or the salt thereof in an amount of 0.3 w/v% or more, and in an amount of 0.5 w/v% or less based on the total volume of the ophthalmic composition. As one example, the ophthalmic composition may include brimonidine or the salt thereof in an amount of 0.3 to 0.5 w/v% or 0.3 to 0.4 w/v%.

In embodiments of the present invention, the ophthalmic composition according to the present invention may include: 0.3 w/v% or more of brimonidine or the salt thereof based on the total volume of the ophthalmic composition; citrate or the hydrate thereof; and trometamol.

In embodiments of the present invention, citrate or the hydrate thereof may be sodium citrate or a hydrate thereof. In the present invention, citrate or the hydrate thereof may be a stabilizer capable of minimizing an occurrence of impurities in the ophthalmic composition. Specifically, in embodiments of the present invention, the ophthalmic composition may include sodium citrate hydrate. In embodiments of the present invention, the ophthalmic composition may include sodium citrate dihydrate (HOC(COONa)(CH2COONa)₂ · 2H₂O).

In embodiments of the present invention, the ophthalmic composition may include citrate or the hydrate thereof in an amount of 0.01 w/v% or more, 0.1 w/v% or more, and in an amount of 10 w/v% or less, 6 w/v% or less, 3 w/v% or less, 1 w/v% or less, 0.8 w/v% or less, 0.6 w/v% or less based on the total volume of the ophthalmic composition. For example, the ophthalmic composition may include citrate in an amount of 0.01 to 10 w/v%, 0.01 to 6 w/v%, 0.01 to 3 w/v%, 0.1 to 3 w/v%, 0.1 to 1 w/v%, 0.1 to 0.6 w/v%, 0.01 to 1 w/v%, 0.01 to 0.8 w/v%, or 0.01 to 0.6 w/v%.

In embodiments of the present invention, trometamol may be a compound having an IUPAC name of 2-amino-2-(hydroxymethyl)propane-1,3-diol. In the present invention, trometamol may adjust a pH of the ophthalmic composition to be more than 7, 7.2 or more, or 7.4 or more.

In embodiments of the present invention, the ophthalmic composition may include trometamol in an amount of 0.01 w/v% or more, 0.1 w/v% or more, and in an amount of 10 w/v% or less, 6 w/v% or less, 3 w/v% or less, 1 w/v% or less, 0.8 w/v% or less, 0.6 w/v% or less based on the total volume of the ophthalmic composition. For example, the ophthalmic composition may include citrate in an amount of 0.01 to 10 w/v%, 0.01 to 6 w/v%, 0.01 to 3 w/v%, 0.1 to 3 w/v%, 0.1 to 1 w/v%, 0.1 to 0.6 w/v%, 0.01 to 1 w/v%, 0.01 to 0.8 w/v%, or 0.01 to 0.6 w/v%. In embodiments of the present invention, a pH of the ophthalmic composition may be 7.2 or more, 7.3 or more, or 7.4 or more, and 9 or less, or 8 or less. For example, the ophthalmic composition may have a pH of 7.2 to 9, 7.2 to 8, 7.3 to 8, or 7.4 to 8.

In embodiments of the present invention, the ophthalmic composition according to the present invention may include: 0.2 w/v% or more of brimonidine or the salt thereof based on the total volume of the ophthalmic composition; citrate or the hydrate thereof; and trometamol, and the pH thereof may be 7.2 or more.

The ophthalmic composition of the present invention may include citrate or the hydrate thereof and trometamol together with brimonidine or the salt thereof, so that the ophthalmic composition may have a pH of more than 7, may have a transparent apperance, may have a reduced occurrence of impurities, and may be stably stored to maintain an osmotic pressure range similar to that of a tear.

In embodiments of the present invention, the ophthalmic composition may include sulfobutylether-β-cyclodextrin (SBE-β-CD) or a salt thereof.

In embodiments of the present invention, the salt of SBE-β-CD may include a metal salt, but is not particularly limited thereto. For example, the salt of SBE-β-CD may be a sodium salt.

In embodiments of the present invention, the ophthalmic composition may include SBE-β-CD or the salt thereof in an amount of 1 w/v% or more, 3 w/v% or more, 4 w/v% or more, 5 w/v% or more, or 6 w/v% or more, and in an amount of 15 w/v% or less, 10 w/v% or less, or 8 w/v% or less. For example, the ophthalmic composition may include SBE-β-CD or the salt thereof in an amount of 1 to 15 w/v%, 1 to 10 w/v%, 3 to 10 w/v%, 4 to 10 w/v%, 5 to 10 w/v%, 4 to 8 w/v%, or 5 to 8 w/v%..

In embodiments of the present invention, the ophthalmic composition may include xanthan gum.

In embodiments of the present invention, the ophthalmic composition may include xanthan gum in an amount of 0.01 w/v% or more, 0.1 w/v% or more, and in an amount of 10 w/v% or less, 6 w/v% or less, 3 w/v% or less, 1 w/v% or less, 0.8% or less, 0.6 w/v% or less. For example, the ophthalmic composition may include xanthan gum in an amount of 0.01 to 10 w/v%, 0.01 to 6 w/v%, 0.01 to 0.6 w/v%, 0.1 to 3 w/v%, 0.1 to 1 w/v%, 0.1 to 0.6 w/v%, or 0.01 to 1 w/v%.

According to the ophthalmic composition of the present invention, it may further include at least one of SBE-β-CD or the salt thereof and xanthan gum in addition to brimonidine or the salt thereof, citrate or the hydrate thereof, and trometamol, thereby stably and sufficiently dissolving brimonidine or the salt thereof in a preparation process, and maintaining a stable state without precipitation and changes in apperance, pH, osmotic pressure, and the like during storage.

In embodiments of the present invention, the ophthalmic composition may include: brimonidine or the salt thereof; citrate or the hydrate thereof; trometamol; and SBE-β-CD. In this case, brimonidine or the salt thereof may be 0.2 w/v% or more based on the total volume of the ophthalmic composition, and the pH of the ophthalmic composition may be 7.2 or more.

In embodiments of the present invention, the ophthalmic composition may include: brimonidine or the salt thereof; citrate or the hydrate thereof; trometamol; and xanthan gum. In this case, brimonidine or the salt thereof may be 0.2 w/v% or more based on the total volume of the ophthalmic composition, and the pH of the ophthalmic composition may be 7.2 or more.

In embodiments of the present invention, the ophthalmic composition may include: brimonidine or the salt thereof; citrate or the hydrate thereof; trometamol; SBE-β-CD or the salt thereof; and xanthan gum. In this case, brimonidine or the salt thereof may be 0.2 w/v% or more based on the total volume of the ophthalmic composition, and the pH of the ophthalmic composition may be 7.2 or more.

In embodiments of the present invention, the ophthalmic composition may include: 0.3 w/v% or more of brimonidine or the salt thereof; citrate or the hydrate thereof; and trometamol. In this case, the ophthalmic composition may include SBE-β-CD or the salt thereof; and/or xanthan gum, and the pH of the ophthalmic composition may be 7.2 or more.

In embodiments of the present invention, the ophthalmic composition may include: 0.3 to 0.4 w/v% of brimonidine or the salt thereof; citrate or the hydrate thereof; and trometamol, and the pH thereof may be 7.2 or more. In this case, the ophthalmic composition may include SBE-β-CD or the salt thereof; and/or xanthan gum.

In embodiments of the present invention, the ophthalmic composition may include: 0.2 w/v% or more of brimonidine or the salt thereof; citrate or the hydrate thereof; trometamol; SBE-β-CD or the salt thereof; and xanthan gum, and the pH thereof may be 7.2 or more, for example, 7.2 to 8.

In embodiments of the present invention, the ophthalmic composition may include: 0.3 w/v% or more of brimonidine or the salt thereof; citrate or the hydrate thereof; trometamol; and SBE-β-CD or the salt thereof; and xanthan gum, and the pH thereof may be 7.4 or more, for example, 7.4 to 8.

In embodiments of the present invention, the ophthalmic composition may include: 0.2 to 0.5 w/v% of brimonidine or the salt thereof; 0.01 to 1 w/v% of citrate or the hydrate thereof; and 0.01 to 1 w/v% of trometamol. In this case, the pH of the ophthalmic composition may be 7.2 to 8.

In embodiments of the present invention, the ophthalmic composition may include: 0.2 to 1 w/v% of brimonidine or the salt thereof; 0.01 to 1 w/v% of citrate or the hydrate thereof; and 0.01 to 1 w/v% of trometamol; 1 to 10 w/v% of SBE-β-CD or the salt thereof; and 0.1 to 0.6 w/v% of xanthan gum. In this case, the pH of the ophthalmic composition may be 7.2 or more, for example, 7.2 to 8.

In embodiments of the present invention, the ophthalmic composition may include: 0.3 to 0.4 w/v% of brimonidine or the salt thereof; 0.01 to 1 w/v% of citrate or the hydrate thereof; and 0.01 to 1 w/v% of trometamol; 1 to 10 w/v% of SBE-β-CD or the salt thereof; and 0.1 to 0.6 w/v% of xanthan gum. In this case, the pH of the ophthalmic composition may be 7.2 or more, for example, 7.2 to 8.

In embodiments of the present invention, the ophthalmic composition may include: 0.2 w/v% of brimonidine or the salt thereof; citrate or the hydrate thereof; and trometamol and may not include sodium hydroxide and hydrochloric acid, and the pH thereof may be 7.2 or more.

In embodiments of the present invention, the ophthalmic composition may include: 0.3 to 0.4 w/v% of brimonidine or the salt thereof; citrate or the hydrate thereof; and trometamol and may not include sodium hydroxide and hydrochloric acid, and the pH thereof may be 7.2 or more, for example, 7.2 to 8.

In embodiments of the present invention, the ophthalmic composition may include an additive such as a buffering agent, a dissolution aid, a sustained-release agent, a thickener, an isotonic agent, etc.

In embodiments of the present invention, the buffering agent may use at least one of acetic acid, phosphoric acid, boric acid, salts thereof and/or hydrates or anhydrides thereof, or the like, and is not limited thereto. The buffering agent may be added in an amount appropriate to maintain an appropriate pH.

In embodiments of the present invention, the ophthalmic composition may include polysorbate 20, polysorbate 80, etc., as a dissolution aid.

In embodiments of the present invention, the ophthalmic composition may include polyvinylpyrrolidone, a cellulose-based compound, etc., as a thickener.

The ophthalmic composition of the present invention may be a solution including water. In the present invention, the "solution" may not include a suspension. In the present invention, the "solution" may not include an emulsion.

The ophthalmic composition of the present invention may not be a suspension. The ophthalmic composition of the present invention may not be an emulsion.

The ophthalmic composition of the present invention may be a solution with a transparent apperance including water. In embodiments of the present invention, water used as an aqueous medium may be sterile purified water, water for injection, etc., suitable for preparing the ophthalmic preparation. In one embodiment, the ophthalmic composition of the present invention may be an aqueous ophthalmic composition including water.

In one embodiment of the present invention, (1) the ophthalmic composition of the present invention may include: 0.2 w/v% or more of brimonidine or the salt thereof; citrate or the hydrate thereof; trometamol, and the pH may be 7.2 or more.

(2) In the ophthalmic composition of above (1), the citrate may be sodium citrate.

(3) In the ophthalmic composition of above (1) or (2), brimonidine or the salt thereof may be 0.2 to 0.5 w/v%.

(4) In the ophthalmic composition of any one of above (1) to (3), the pH may be 7.2 to 8.

(5) In the ophthalmic composition of any one of above (1) to (4), the ophthalmic composition may include sulfobutylether-β-cyclodextrin (SBE-β-CD) or the salt thereof; and xanthan gum.

(6) In the ophthalmic composition of above (5), SBE-β-CD or the salt thereof may be included in an amount of 1 to 15 w/v% and xanthan gum may be included in an amount of 0.01 to 1 w/v%.

(7) In the ophthalmic composition of any one of above (1) to (6), the ophthalmic composition may be a transparent solution including water.

(8) In the ophthalmic composition of any one of above (1) to (7), the ophthalmic composition may include at least one additive selected from a buffering agent, a dissolution aid, a sustained-release agent, and a thickener.

(9) In the ophthalmic composition of any one of above (1) to (8), the ophthalmic composition may not include sodium hydroxide and hydrochloric acid and the pH thereof may be 7.2 to 8.

(10) In the ophthalmic composition of any one of above (1) to (9), the ophthalmic composition may be for preventing, ameliorating or treating glaucoma and/or ocular hypertension.

In embodiments of the present invention, a method for preventing, ameliorating or treating glaucoma and/or ocular hypertension according to the present invention may include administering an ophthalmic composition of any one of above (1) to (9) into a subject in need thereof.

In one embodiment of the present invention, the present invention may provide a use of the ophthalmic composition of any one of above (1) to (9) for preventing, ameliorating or treating glaucoma and/or ocular hypertension.

In one embodiment of the present invention, the present invention may provide a use of the ophthalmic composition of any one of above (1) to (9) for preparing a medication for preventing, ameliorating or treating glaucoma and/or ocular hypertension.

In embodiments of the present invention, an osmotic pressure of the ophthalmic composition may be 250 to 400 mOsmol/kg. For example, the osmotic pressure of the ophthalmic composition of the present invention may be 270 to 330 mOsmol/kg.

In embodiments of the present invention, when the ophthalmic composition is stored at a temperature of 70°C, the amount of individual unknown impurities produced may be less than 0.1%, 0.05% or less for seven days.

In embodiments of the present invention, when the ophthalmic composition is stored at a temperature of 70°C, the amount of individual unknown impurities produced may be reduced by 5% or more, 10% or more, 20% or more, 30% or more, 40% or more, or 50% or more.

In embodiments of the present invention, the ophthalmic composition may be a solution that is continuously transparent and stable for at least three months when stored at a temperature of 20°C to 40°C.

In embodiments of the present invention, the ophthalmic composition may be a solution that is continuously transparent and stable for at least two weeks when stored at a temperature of 70°C.

In embodiments of the present invention, the ophthalmic composition may include brimonidine or the salt thereof as an active ingredient, and may show a pharmacological activity exhibited by brimonidine or the salt thereof. Specifically, the ophthalmic composition may be advantageously used for preventing, ameliorating or treating glaucoma and/or ocular hypertension.

In embodiments of the present invention, the ophthalmic composition may further include an active ingredient in addition to brimonidine or the salt thereof. Examples of the additional active ingredient may include latanoprost, bimatoprost, travoprost, tafluprost, netarsudil, carteolol, timoptic, timolol, betaloxol, levobunolol, metipranolol, brinzolamide, dorzolamide, acetazolamide, methazolamide, or a pharmaceutically acceptable salt of each thereof, each of which may be used alone or at least two of which may be used in combination with brimonidine or the salt thereof.

In embodiments of the present invention, the ophthalmic composition may be disposable or for multi-use, and specifically may be disposable. When the ophthalmic composition is for multi-use, the composition may further include a preservative. The preservative may be a quaternary ammonium compound such as benzalkonium chloride, polyquaternium-1 (e.g., polyquads), etc.; a guanidine-based compound such as chlorohexidine, etc.; chlorobutanol; a mercury preservative, etc.

In embodiments of the present invention, the ophthalmic composition may be administered once to three times a day. For example, the ophthalmic composition may be administered once, twice, or three times a day.

The ophthalmic composition of the present invention may include brimonidine stably dissolved therein, and thus may be provided as an aqueous solution having a transparent apperance. In addition, the ophthalmic composition of the present invention may not cause precipitation during storage, and may be maintained in a stable state without a change in the properties thereof such as color during storage. In addition, the ophthalmic composition may have excellent stability in that physical and chemical properties such as a content of active ingredients, pH, etc., are well maintained and a small amount of impurities are produced. Thus, the ophthalmic composition of the present invention may be maintained in a stable state for a long period of time, and thus may be safely administered to an eye and may exhibit an excellent therapeutic effect without side effects.

A method for preparing an ophthalmic composition according to the present invention may include preparing a mixed solution containing brimonidine or a salt thereof, citrate or a hydrate thereof, and trometamol.

In embodiments of the present invention, the preparing of the mixed solution may include: preparing a first solution containing brimonidine or a salt thereof, citrate or a hydrate thereof, and water;
preparing a second solution containing trometamol and water; and
mixing the first and second solutions. In the preparing of the mixed solution, citrate or the hydrate thereof may be included in the second solution. In the preparing of the mixed solution, a portion of a total content of trometamol included in the ophthalmic composition according to the present invention may be included in the preparing of the first solution.

In embodiments of the present invention, the mixed solution may be a solution subjected to a sterilization process. In one embodiment, after the sterilization process is performed on the first solution and the second solution, they may be mixed to prepare the mixed solution.

In embodiments of the present invention, the first solution, the second solution, and the mixed solution in the preparing of the mixed solution may each independently further include SBE-β-CD or a salt thereof, and/or xanthan gum.

In embodiments of the present invention, the first solution, the second solution, and the mixed solution in the preparing of the mixed solution may each independently further include an additive such as a buffering agent, a pH adjusting agent, a dissolution aid, a sustained-release agent, a thickener, an isotonic agent, etc.

In embodiments of the present invention, mixing an additional active ingredient in addition to brimonidine or the salt thereof may be further included in the preparing of the mixed solution.

The method for preparing the ophthalmic composition according to the present invention may include: adding the pH adjusting agent in the preparing of the first solution and/or adding the pH adjusting agent to the mixed solution. In this case, the pH adjusting agent may be hydrochloric acid or sodium hydroxide. For example, in embodiments of the present invention, hydrochloric acid may be added to dissolve brimonidine in the preparing of the first solution, and/or hydrochloric acid or sodium hydroxide may be added after mixing the first solution and the second solution in the preparing of the mixed solution.

The ophthalmic composition of the present invention may be for preventing, ameliorating or treating glaucoma and/or ocular hypertension.

The present invention may provide a method for preventing, ameliorating or treating glaucoma and/or ocular hypertension, the method including administering an effective amount of the ophthalmic composition of the present invention into a subject in need thereof.

The present invention may provide a use of the ophthalmic composition of the present invention for preventing, ameliorating or treating glaucoma and/or ocular hypertension.

The present invention may provide a use of the ophthalmic composition of the present invention for preparing a medication for preventing, ameliorating or treating glaucoma and/or ocular hypertension.

In the method for preparing the ophthalmic composition according to the present invention, brimonidine or the salt thereof, citrate or the hydrate thereof and trometamol, and SBE-β-CD or the salt thereof, and xanthan gum, furthermore, additives and additional active ingredients, which are further added, may be substantially the same as those described above, and thus a redundant detailed description will be omitted.

### Advantageous Effects

The ophthalmic composition of the present invention may include brimonidine stably dissolved therein, and thus may be provided as an aqueous solution having a transparent apperance.

In addition, the ophthalmic composition of the present invention may not cause precipitation during storage, and may be maintained in a stable state without a change in the properties thereof such as color during storage.

In addition, the ophthalmic composition may have excellent stability in that physical and chemical properties such as a content of active ingredients, pH, osmotic pressure, etc., are well maintained and a small amount of impurities are generated.

Thus, the ophthalmic composition of the present invention may be safely administered into an eye for a long period of time and may be maintained in a stable state, thereby exhibiting an excellent therapeutic effect without side effects.

### Brief Description of Drawings

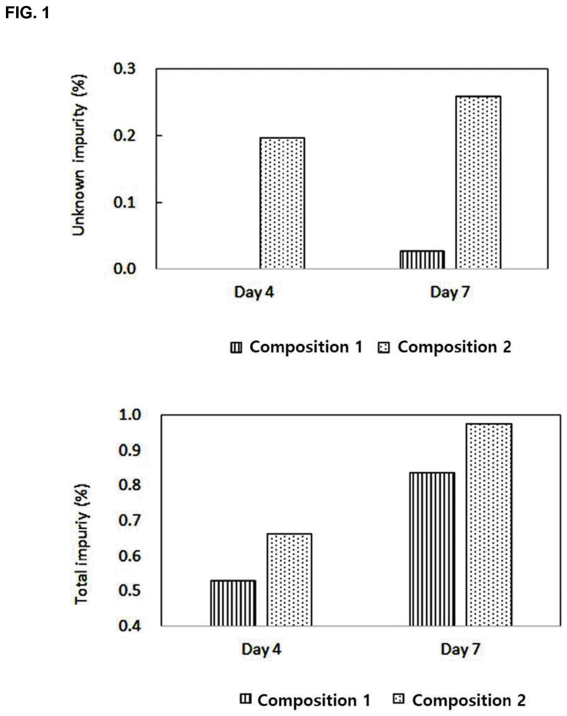

FIG. 1 is a drawing showing the results of experimental example 2.

### Mode for Invention

Hereinafter, the present invention will be described in more detail through experimental examples. These experimental examples are provided only for the purpose of illustrating the present invention, and thus it will be apparent to those skilled in the art that the scope of the present invention is not limited thereto.

### Preparation Example 1: Preparation of ophthalmic compositions 1 to 4

Compositions 1 to 4 were prepared according to the ingredients and contents described in table 1 below.

Specifically, brimonidine tartrate, sodium citrate dihydrate, SBE-β-CD sodium, and trometamol were dissolved in water and subjected to a filter sterilization process to provide a first solution. Separately, xanthan gum and trometamol were dissolved in water and subjected to a high-temperature and high-pressure sterilization process to provide a second solution. Composition 1 was prepared by mixing the first solution and the second solution.

Composition 2 was prepared in the same manner as in composition 1, except for sodium citrate dihydrate.

Composition 3 was prepared by adjusting a pH with sodium hydroxide or hydrochloric acid without trometamol.

Composition 4 was prepared by adjusting the pH with sodium hydroxide or hydrochloric acid without sodium citrate dihydrate and trometamol.

The pH of the prepared compositions 1 to 4 was about 7.6.

**[Table 1]**

| Ingredient | Content (w/v%) | | | |
|---|---|---|---|---|
| | Composition 1 | Composition 2 | Composition 3 | Composition 4 |
| Brimonidine tartrate | 0.4 | 0.4 | 0.4 | 0.4 |
| SBE-β-CD sodium | 6 | 6 | 6 | 6 |
| Xanthan gum | 0.2 | 0.2 | 0.2 | 0.2 |
| Sodium citrate dihydrate | 0.3 | - | 0.3 | - |
| Trometamol | 0.2 | 0.2 | - | - |
| Sodium hydroxide or hydrochloric acid | - | - | Appropriate amount | Appropriate amount |
| Water (water for injection) | Appropriate amount | Appropriate amount | Appropriate amount | Appropriate amount |

### Experimental Example 1: Stability evaluation 1 - Property and pH

Compositions 1 to 4 were all prepared as a transparent solution.

In addition, each of compositions 1 to 4 was stored at 70°C for 10 days, and a change in the pH was measured from days 0 to 10.

As a result, it was confirmed that composition 1 maintains a transparent apperance without precipitation, while the pH on day 10 is 7.5 at the same time, and thus there is substantially no change in pH.

On the contrary, in the case of compositions 3 and 4, it was confirmed that the pH is significantly changed to the range of about 7.1 to 7.2. In particular, in the case of compositions 3 and 4, it was confirmed that the pH is rapidly changed to the level of about 7.2 to 7.3 just on day 4.

Through this, it can be seen that composition 1 containing both citrate and trometamol of the present invention has very excellent properties and pH stability.

### Experimental Example 2: Stability evaluation 2 - Amount of impurities produced

The compositions were stored at 70°C for one week, and then the amounts of unknown impurities and total impurities produced on days 4 and 7 were measured by liquid chromatography.

As a result, it can be confirmed that composition 1 does not have the amount of unknown impurities produced on day 4 and the amount is only about less than 0.1% on day 7, which falls on about 0.05% or less (see FIG. 1). On the contrary, in the case of composition 2, it can be confirmed that the amount of unknown impurities produced is already about 0.2% on day 4 and increases up to about 0.25% on day 7. In addition, it can be confirmed that the amount of total impurities produced from composition 2 is more than that of composition 1 (about 16% increase).

Through this, it can be seen that the composition containing both citrate and trometamol of the present invention has very excellent stability with a less amount of impurities produced.

### Preparation Example 2: Preparation of ophthalmic compositions 5 to 7

Compositions 5 to 7 were prepared in the same manner as in the preparation method of composition 1 according to the ingredients and contents shown in table 2 below, but the pH was adjusted with sodium hydroxide or hydrochloric acid, if necessary. Specifically, the pH was adjusted to about 7.6 by further adding hydrochloric acid to dissolve brimonidine therein at the time of preparing the first solution, or using hydrochloric acid or sodium hydroxide after mixing the first solution and the second solution.

**[Table 2]**

| Ingredient | Content (w/v%) | | |
|---|---|---|---|
| | Composition 5 | Composition 6 | Composition 7 |
| Brimonidine tartrate | 0.2 | 0.5 | 0.4 |
| SBE-β-CD sodium | 1 | 10 | 4 |
| Xanthan gum | 0.01 | 0.6 | 0.1 |
| Sodium citrate dihydrate | 0.01 | 0.1 | 0.6 |
| Trometamol | 0.01 | 0.6 | 0.1 |
| Sodium hydroxide or hydrochloric acid | Appropriate amount | Appropriate amount | Appropriate amount |
| Water (water for injection) | Appropriate amount | Appropriate amount | Appropriate amount |

### Experimental Example 3: Stability evaluation 3 - Property and pH

While the compositions 5 to 7 were stored at 70°C for 14 days, the apperance thereof were observed and the pH thereof was measured.

As a result, it was confirmed that all of compositions 5 to 7 maintain a transparent solution state and there is no change in apperance. In addition, it was confirmed that the pHs of compositions 5 to 7 are all stably maintained (see table 3).

**[Table 3]**

| | Composition 5 | Composition 6 | Composition 7 |
|---|---|---|---|
| Day 0 | 7.66 | 7.59 | 7.57 |
| Day 10 | 7.59 | 7.30 | 7.44 |
| Day 14 | 7.63 | 7.30 | 7.45 |

Through this, it can be seen that the composition containing both citrate and trometamol of the present invention has very excellent properties and pH stability.

### Preparation Example 3: Preparation of ophthalmic compositions 8 to 10

Compositions 8 to 10 were prepared in the same manner as in the preparation method of composition 1 according to the ingredients and contents shown in table 4 below, but the pH was adjusted with sodium hydroxide or hydrochloric acid, if necessary. Specifically, the pH was adjusted to about 7.6 by further adding hydrochloric acid to dissolve brimonidine therein at the time of preparing the first solution, or using hydrochloric acid or sodium hydroxide after mixing the first solution and the second solution.

**[Table 4]**

| Ingredient | Content (w/v%) | | |
|---|---|---|---|
| | Composition 8 | Composition 9 | Composition 10 |
| Brimonidine tartrate | 0.4 | 0.2 | 0.5 |
| SBE-β-CD sodium | 4 | 1 | 10 |
| Xanthan gum | 0.1 | 0.01 | 0.6 |
| Sodium citrate dihydrate | 0.6 | 0.2 | 0.2 |
| Trometamol | 0.2 | 0.2 | 0.6 |
| Sodium hydroxide or hydrochloric acid | Appropriate amount | Appropriate amount | Appropriate amount |
| Water (water for injection) | Appropriate amount | Appropriate amount | Appropriate amount |

### Experimental Example 4: Stability evaluation 4 - Content of active ingredient and amount of impurities produced

While the compositions 8 to 10 were stored at 70°C for 14 days, the content of active ingredients and the amount of unknown impurities produced were measured by liquid chromatography.

As a result, it was confirmed that the content (%) of the active ingredients is stably maintained in all of compositions 8 to 10 (see table 5), and it was confirmed that the amount of unknown impurities produced therefrom is very small (see table 6).

**[Table 5]**

| | Composition 8 | Composition 9 | Composition 10 |
|---|---|---|---|
| Day 0 | 101.6 | 102.2 | 100.8 |
| Day 7 | 99.6 | 100.8 | 100.7 |
| Day 14 | 98.5 | 99.4 | 98.7 |

**[Table 6]**

| | Composition 8 | Composition 9 | Composition 10 |
|---|---|---|---|
| Day 0 | 0.00 | 0.00 | 0.00 |
| Day 7 | 0.03 | 0.03 | 0.02 |
| Day 14 | 0.05 | 0.06 | 0.04 |

Through this, it can be seen that the composition containing citrate and trometamol of the present invention has very excellent stability.

While specific portions of the present invention have been described in detail above, it is apparent to those skilled in the art that such detailed descriptions are set forth to illustrate exemplary embodiments only, but are not construed to limit the scope of the present invention. Thus, it should be understood that the substantial scope of the present invention is defined by the accompanying claims and equivalents thereto.

## Claims

1. An ophthalmic composition comprising:
0.2 w/v% or more of brimonidine or a salt thereof;
citrate or a hydrate thereof; and
trometamol,
wherein a pH is 7.2 or more.

2. The ophthalmic composition of claim 1, wherein the citrate is sodium citrate.

3. The ophthalmic composition of claim 1, wherein brimonidine or the salt thereof is 0.2 to 0.5 w/v%.

4. The ophthalmic composition of claim 1, wherein the pH is 7.2 to 8.

5. The ophthalmic composition of claim 1, wherein the ophthalmic composition comprises sulfobutylether-β-cyclodextrin (SBE-β-CD) or a salt thereof; and xanthan gum.

6. The ophthalmic composition of claim 5, wherein SBE-β-CD or the salt thereof is comprised in an amount of 1 to 15 w/v% and xanthan gum is comprised in an amount of 0.01 to 1 w/v%.

7. The ophthalmic composition of claim 1, wherein the ophthalmic composition is a transparent solution including water.

8. The ophthalmic composition of claim 1, wherein the ophthalmic composition comprises at least one additive selected from a buffering agent, a dissolution aid, a sustained-release agent, and a thickener.

9. The ophthalmic composition of claim 1, wherein the ophthalmic composition does not comprise sodium hydroxide and hydrochloric acid and the pH thereof is 7.2 to 8.

10. The ophthalmic composition of any one of claims 1 to 9, wherein the ophthalmic composition is for preventing, ameliorating or treating glaucoma and/or ocular hypertension.

11. A method for preventing, ameliorating or treating glaucoma and/or ocular hypertension, the method comprising administering an ophthalmic composition of any one of claims 1 to 9 into a subject in need thereof.

12. A use of an ophthalmic composition of any one of claims 1 to 9 for preventing, ameliorating or treating glaucoma and/or ocular hypertension.

13. A use of an ophthalmic composition of any one of claims 1 to 9 for preparing a medication for preventing, ameliorating or treating glaucoma and/or ocular hypertension.
